# EUROPEAN PATENT SPECIFICATION

(11) **EP 1 646 865 B1**
(45) Date of publication and mention of the grant of the patent: **12.09.2007**
(21) Application number: 04740635.0
(22) Date of filing: 05.07.2004
(51) Int. Cl.: G01N 33/50

(54) **METHOD FOR TESTING GHRELIN RELEASE**
VERFAHREN ZUM TESTEN DER GHRELIN-FREISETZUNG
METHODE PERMETTANT DE TESTER UNE LIBERATION DE GHRELINE

(30) Priority: 17.07.2003 EP 03254472
(43) Date of publication of application: 19.04.2006
(73) Proprietor: UNILEVER N.V., 3013 AL Rotterdam (NL); UNILEVER PLC, London EC4P 4BQ (GB)
(72) Inventor: GERHARDT, Cinderella Christina, Unilever R&D Vlaardingen, NL-3133 AT Vlaardingen (NL); TASKER, Maria Catherine, Unilever R&D Vlaardingen, NL-3133 AT Vlaardingen (NL)
(74) Representative: Wurfbain, Gilles L.
(86) International application number: PCT/EP2004/007297
(87) International publication number: WO 2005/015208

(56) References cited:
- WO-A-02/090387
- US-B1- 6 432 654
- JI JIAFU ET AL: "Comprehensive Analysis of the Gene Expression Profiles in Human Gastric Cancer Cell Lines" ONCOGENE, vol. 21, no. 42, 19 September 2002 (2002-09-19), pages 6549-6556, XP002261627 ISSN: 0950-9232
- KARBONITS M., ET AL: "The Expression of the Growth Hormone Secretagogue Receptor Ligand Ghrelin in Normal and Abnormal Human Pituitary and Other Neuroendocrine Tumors" J. CLIN. ENDOCRINOL. METAB., vol. 86, no. 2, 28 October 2000 (2000-10-28), - 28 October 2000 (2000-10-28) pages 881-887, XP002261629
- RINDI GUIDO ET AL: "Ghrelin expression in gut endocrine growths" HISTOCHEMISTRY AND CELL BIOLOGY, vol. 117, no. 6, June 2002 (2002-06), pages 521-525, XP008024735 ISSN: 0948-6143 cited in the application
- VOLANTE M., ET AL: "Expression of Ghrelin and of the GH Secretagogue Receptor by Pancreatic Islet Cells and Related Endocrine Tumors" J. CLIN. ENDOCRINOL. METAB., vol. 87, no. 3, 1 March 2002 (2002-03-01), - 31 March 2003 (2003-03-31) pages 1300-1308, XP002261628

## Description

### Field of the invention

The present invention relates to the use of a cell line, preferably RF-1 or RF-48, derived from a human gastric adenocarcinoma of human caecum, to measure regulation of expression and/or secretion of a peptide called ghrelin. Ghrelin, the length of which is 28 amino acids, is the ligand for the growth hormone receptor. Ghrelin is known to exist in at least two forms:
1) n-octanoyl ghrelin in which the third serine residue is n-octanoylated and 2) des-n-octanoyl ghrelin in which the n-octanoyl group is removed. These two forms of ghrelin have different physiological functions.

### Background of the invention

Although ghrelin has only been identified in 1999, more than 200 papers and articles on the substance have already been published, indicating the importance of this peptide hormone. See for example WO02/090387; US6432652; Rindi et al., 2002, "Ghrelin expression in gut endocrine growth", Histochem Cell Biol, 117: 521-525; Volante et al., 2002, "Expression of Grehlin and of the GH Secretagogue Receptor by Pancreatic inlet cells and related endocrine Tumors", Journal of Clinical Endocrinology & Metabolism 87(3): 1300-1313; Korbonits et al., 2001, "The Expression of the Growth Hormone Secretagogue Receptor Ligand Ghrelin in Normal and Abnormal Human Pituitary and other Neuroendocrine Tumors", Journal of Clinical Endocrinology & Metabolism, Vol. 86, No. 2, 881-887.

Ji et al., 2002, "Comprehensive analysis of the gene expression profiles in human gastric cancer cell lines" discloses that cell lines RF1 and RF48 are found in gastric ardenocarcinova cells. The histiogenetic origin of these cell lines are redefined by the authors as B-cell lymphomas. No disclosure is given of the ability or use of these cells to express Grehlin.

Ghrelin is the first identified peripheral hormone signaling appetite. People who were given ghrelin increased their appetite resulting in up to one third more food intake than control subjects. This earned ghrelin the name, the "hunger hormone".

In addition to stimulating food intake, ghrelin levels drop once an individual starts eating. Consequently, it is believed that ghrelin acts as a trigger to start food intake. Furthermore, data have been reported showing that ghrelin levels do not fall after eating in obese individuals which suggests that this trigger is not reset in such individuals.

It has also been suggested in the past that ghrelin could affect our body's internal clock, thus making us feel hungry at certain times during the day.

Many people all over the world suffer from disturbances in their appetite and, as a consequence, their food intake. When energy intake and energy expenditure are in imbalance, body weight changes which can have severe physiological consequences. On one hand there is a large and rapidly growing group of overweight or obese people. In most cases, their excessive body weight is a consequence of increased food intake in combination with decreased energy expenditure. On the other hand there is a group of people suffering from decreased appetite and low body weight. This can be due to illness such as cancer or anorexia, but is also observed in many elderly people.

Decreasing the level of ghrelin in an individual could inhibit hunger feelings and thereby help people, particularly overweight or obese people, to decrease their food intake and lose weight. In contrast, increasing the level of ghrelin could boost appetite and stimulate food intake in ill or old people, generally improving their health and wellbeing.

The properties and characteristics of ghrelin could pave the way to the formulation of a drug that either blocks or increases the level and/or activity of this hormone.

Modulation of ghrelin levels and/or activity could also be used as a basis for developing functional ingredients for food products that modulate feelings of satiety or appetite in humans or animals.

Discovery of such drugs or ingredients would be facilitated by an in vitro model system that mimics the production pathway of ghrelin.

Ghrelin is mainly produced in the stomach, but it also exists in parts of the brain that controls the appetite.

Ghrelin has furthermore been shown to occur in endocrine tumors of the human stomach, pancreas, intestine and lungs (G. Rindi et al (Histochem Cell Biol. 2002 June, 117(6), 521-5). Regulation of the expression, synthesis, and secretion of ghrelin is however still largely unknown.

It is an object of the present invention to find a screening method for assessing the effect of test compounds on the level and activity of ghrelin.

It is another object of the invention that this screening method is simple and reproducible.

It is a further object of the invention to provide a method for analysing the secretion of both forms of ghrelin, in particular for examining the endogenous molecules that regulate their synthesis and secretion.

It is a still further object of the invention to provide a method for analysing regulation of ghrelin gene expression.

It is also an object of the invention that this screening method can be used for estimating the effect on food intake of a variety of test compounds, which were found to suppress or stimulate the release of ghrelin in vitro.

We have now surprisingly found that these objects can be achieved by a cellular model for studying the level of ghrelin production. This model comprises a cell line derived from a human gastric adenocarcinoma, which can release ghrelin upon growth in a suitable medium.

### Definition of the invention

Accordingly, in a first aspect, the present invention provides a model to study the (regulation of) expression, synthesis and/or secretion of ghrelin, comprising in vitro culture of a cell line derived from a gastric adenocarcinoma, said cell line being capable of producing ghrelin, and said model also comprising a medium for growing said cell line. The model of the invention is particularly useful for finding compounds which can modulate appetite in humans. However, the model can also be used for screening compounds that can modulate appetite in animals, and that could be added to functional pet food or feed.

In a second aspect, the present invention provides a method for assessing the (regulation of) expression, synthesis and/or secretion of ghrelin, wherein a cell line derived from a gastric adenocarcinoma and capable of producing ghrelin when grown in a suitable medium, is grown in such medium. In the method of the invention, the cell line is preferably incubated with a test compound, and the effect of the test compound on the production of ghrelin is preferably measured.

In a third aspect, the present invention provides the use of a cell line derived from a gastric adenocarcinoma that is capable of producing ghrelin when grown in a suitable medium, for assessing the (regulation of) ghrelin expression, synthesis and/or release in vitro.

### Detailed description of the invention.

We have found that the cell lines used in the present invention are suitable for measuring expression, synthesis and/or secretion of ghrelin. Furthermore, the model and method of the invention appeared to be suitable for screening a large number of test compounds with regard to their effect on ghrelin expression and/or secretion. This effect can either be a stimulating effect or a blocking effect.

It has also been found that the method of the invention is suitable for screening simultaneously a number of test compounds that are expected to modulate (i.e. stimulate or block) ghrelin expression and/or secretion, at various concentrations. Furthermore, application of the method of the invention can provide an estimation of the effect of various test compounds on food intake in humans or animals thereby decreasing the complexity of in vivo feeding trials.

The method and model of the invention were found to be particularly suitable for use with a cell line derived from a human gastric adenocarcinoma. Preferably, the cell line is selected from RF-1 having ATCC number CRL-1864 and RF-48 having ATCC number CRL-1863.

Preferably, the medium for growing the cell line used in the present invention is Leibovitz's L15 containing 10% (vol/vol) foetal bovine serum and 2 mM L-glutamine, and the cell line is desirably grown in said medium at a temperature of 37° C and in the absence of CO₂.

The medium for growing the cell line is effectively changed at least every 4 days, preferably every 3-4 days, until cell confluence is achieved. Subsequently, the cells are suitably grown and plated in 24-well cultures plates whereby each well may contain 1x 10⁴ to 5x 10⁶ cells. The cells could also be grown in cultures plates containing different numbers of wells, such as 48 wells or 96 wells.

Preferably, these plates are stored under the same incubation conditions as those used for growing the cell line.

In the screening method of the invention, the cells are subsequently exposed to a variety of test compounds, whereby ghrelin release may effectively be measured using an immunoassay kit. Preferably, for each treatment with a test compound three samples were prepared. In other words, the effect on ghrelin release by each type of test compound is desirably measured in triplicate. In order to be able to make reliable comparisons it is also desirable to prepare a control sample that does not contain any test compound, in triplicate.

In a particular aspect of the invention a method is provided wherein the cell line is plated and grown in a culture plate after achieving cell confluence, the plate is stored under the same incubation conditions as those used for growing the cell line, and ghrelin production is measured using an immunoassay kit.

In the screening method of the invention the effect of the test compounds on ghrelin release may effectively be measured as follows. The cultures plates are stored as described above and, after 0.5 -3 hours incubation, a sample is taken from each well to measure ghrelin release. Each sample is centrifuged at 1000-5000 rpm during 1-10 minutes to remove the cells from the sample. The supernatant containing the medium, the test compound and, additionally, any ghrelin secreted is then transferred to a separate tube. The thus-formed supernatant samples are suitably frozen, e.g. at -80 °C, for subsequent analysis of ghrelin secretion. The supernatant samples may also not be frozen and directly used in the measuring device.

Ghrelin release can be suitably measured using an immunoassay kit. This kit may be a commercial enzyme immunoassay -EIA- kit (from Phoenix Pharmaceutical, Belmont, California, USA). Alternatively, two types of radioactive immunoassay (RIA) kits (from Linco Research Inc., Missouri, USA) are available with which either the level of n-octanoyl ghrelin or the total level of ghrelin released (i.e. both forms, see above) can be measured.

The cells according to the present invention can additionally be used in a screening method for assessing the effect of test compounds on ghrelin gene expression, preferably by means of quantitative RT-PCR (Reverse Transcription -Polymerase Chain Reaction). The cells can furthermore be used to assess endogenous molecules that regulate ghrelin expression, synthesis or secretion. Finally, the cells of the present invention can be used in a method to test the effect of compounds that may modulate ghrelin activity.

The following examples are intend to illustrate the invention. Further examples with the scope of the invention will be apparent to the person skilled in the art.

### Examples

The following compounds were tested with regard to their ability to stimulate ghrelin release:
- Tyr(SO3H]27)Cholecystokinin fragment 26-33 Amide (referred to as CCK); and
- Gastrin I human (referred to as Gastrin).

These test compounds were made as 100X stock solutions by dissolving them in pure ethanol, as a vehicle. The test compounds were then diluted 1/100 in the Leibovitz L-15 medium containing 0.5% foetal bovine serum (FBS).

Since the effect caused by these test compounds on ghrelin release by a RF-48 cell line was tested, RF-48 cells were grown during incubation at 37° C in Leibovitz's L15 medium with 2 mM L-glutamine and containing 10% (vol/vol) FBS in the absence of CO₂.

After cell confluence was obtained, the cells were plated in 24-well cultures plates (1x 10⁵ cells/well). Several wells were subsequently exposed to one of the test compounds as prepared above. For each type and concentration of test compound tested, a series of three tests was carried out.

After 1 hour of incubation of the thus-filled wells containing both the cells and the test compound at the same conditions as those applied during growing of the RF-48 cells, a sample was taken from each well to measure ghrelin release.

Each sample was centrifuged at 3000 rpm to remove the cells from the sample and the supernatant (containing the ghrelin formed as well as the medium and the test compound) was transferred to a separate tube. The ghrelin release was measured using a commercial enzyme immunoassay kit (from Phoenix Pharmaceuticals, Belmont, California, USA).

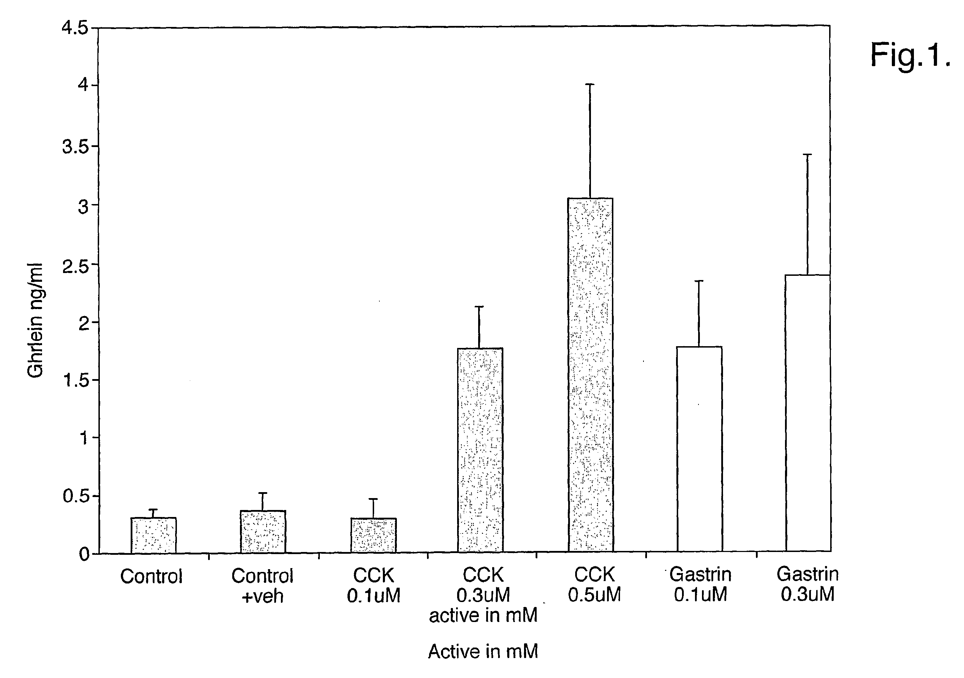

The results are shown in Figure 1, showing the amount of ghrelin released from RF-48 cells as a function of the type and concentration of the test compound tested.

In Figure 1, the concentration of the test compound is shown in micromols per litre (10⁻⁶ M) and the concentration of the ghrelin formed in the tested samples is shown in nanograms/ml.

Figure 1 also provides the results of a control experiment, showing the ghrelin release when no test compound is present in the sample containing the RF-48 cells. In addition, the results of a second control experiment are provided -indicated as "Control +veh" -, also showing ghrelin release when no test compound is present in the sample containing the RF-48 cells but whereby 1% of pure ethanol has been added to said sample.

It can be clearly noticed that both CCK and Gastrin stimulate the release of ghrelin. It can also be seen that both control experiments show similar levels of ghrelin release, which clearly indicates that the ethanol vehicle does not stimulate ghrelin release.

## Claims

1. A method for assessing the (regulation of) expression, synthesis and/or secretion of ghrelin, wherein a cell line derived from a gastric adenocarcinoma and capable of producing ghrelin when grown in a suitable medium, is grown in such medium and wherein the effect of a test compound on ghrelin production is measured.

2. A method according to claim 1, wherein the cell line is selected from RF-1 having ATCC number CRL-1864 and RF-48 having ATCC number CRL-1863.

3. A method according to either one of claims 1 or 2, wherein the medium is Leibovitz's L15 containing 10% (vol/vol) foetal bovine serum and 2 mM L-glutamine, and wherein the cell line is grown at a temperature of 37° C in the absence of CO2.

4. A method according to any one of claims 1 to 3, wherein the medium is changed at least every 4 days.

5. A method according to any one of claims 1 to 4, wherein the cell line is plated and grown in a culture plate after achieving cell confluence, wherein the plate is stored under the same incubation conditions as those used for growing the cell line, and wherein ghrelin production is measured using an immunoassay kit.

6. Method according to any one of claims 1 to 5, wherein the cell line is used to study ghrelin gene expression, preferably by means of quantitative RT-PCR.

7. A method according to any one of claims 1 to 6, wherein the cell line is exposed to a variety of test compounds.

8. A method according to any one of claims 1 to 7, wherein the method is a method for developing drugs or functional ingredients for food products that modulate feelings of satiety or appetite in humans or animals.

9. The use of a cell line derived from a gastric adenocarcinoma that is capable of producing ghrelin when grown in a suitable medium, for assessing the (regulation of) ghrelin expression, synthesis and/or release in vitro.

10. The use according to claim 9, wherein the cell line is selected from RF-1 having ATCC number CRL-1864 and RF-48 having ATCC number CRL-1863.

## Patentansprüche

1. Verfahren zum Bewerten der (Steuerung von) Expression, Synthese und/oder Sekretion von Ghrelin, worin eine Zelllinie, welche von einem gastrischen Adenokarzinom abgeleitet und in der Lage ist, Ghrelin herzustellen, wenn sie in einem geeigneten Medium gezüchtet wird, in solch einem Medium gezüchtet wird, und worin die Wirkung einer Testverbindung auf Ghrelinherstellung gemessen wird.

2. Verfahren gemäß Anspruch 1, worin die Zelllinie ausgewählt wird aus RF-1 mit ATCC-Nummer CRL-1864 und RF-48 mit ATCC-Nummer CRL-1863.

3. Verfahren gemäß einem von Ansprüchen 1 oder 2, worin das Medium Leibovitz L15 ist, welches 10% (Vol./Vol.) fötales Rinderserum und 2 mM L-Glutamin enthält, und worin die Zelllinie bei einer Temperatur von 37°C in der Abwesenheit von CO2 gezüchtet wird.

4. Verfahren gemäß einem von Ansprüchen 1 bis 3, worin das Medium mindestens alle 4 Tage gewechselt wird.

5. Verfahren gemäß einem von Ansprüchen 1 bis 4, worin die Zelllinie nach Erreichen von Zellkonfluenz in einer Kulturschale plattiert und gezüchtet wird, worin die Schale unter den gleichen Inkubationsbedingungen gelagert wird wie jenen, welche zum Züchten der Zelllinie verwendet werden, und worin Ghrelinherstellung unter Verwendung eines Immuntest-Kits gemessen wird.

6. Verfahren gemäß einem von Ansprüchen 1 bis 5, worin die Zelllinie verwendet wird, um Ghrelin-Genexpression vorzugsweise mittels quantitativer RT-PCR zu untersuchen.

7. Verfahren gemäß einem von Ansprüchen 1 bis 6, worin die Zelllinie einer Vielfalt von Testverbindungen ausgesetzt wird.

8. Verfahren gemäß einem von Ansprüchen 1 bis 7, worin das Verfahren ein Verfahren zum Entwickeln von Arzneien oder funktionalen Inhaltsstoffen für Nahrungsmittelprodukte ist, die Gefühle von Sattheit oder Appetit in Menschen oder Tieren modulieren.

9. Verwendung einer Zelllinie, abgeleitet von einem gastrischen Adenokarzinom, die in der Lage ist, Ghrelin herzustellen, wenn sie in einem geeigneten Medium gezüchtet wird, zum Bewerten der (Steuerung von) Ghrelinexpression, -synthese und/oder -abgabe in vitro.

10. Verwendung gemäß Anspruch 9, worin die Zelllinie ausgewählt wird aus RF-1 mit ATCC-Nummer CRL-1864 und RF-48 mit ATCC-Nummer CRL-1863.

## Revendications

1. Méthode pour l'évaluation de (la régulation de) l'expression, de la synthèse et/ou de la sécrétion de la ghréline, dans laquelle une lignée cellulaire dérivée d'un adénocarcinome gastrique et capable de produire de la ghréline lorsqu'elle est cultivée dans un milieu adapté, est cultivée dans un milieu tel et dans laquelle l'effet d'un composé test sur la production de ghréline est mesuré.

2. Méthode selon la revendication 1, dans laquelle la lignée cellulaire est sélectionnée parmi la RF-1 ayant le numéro ATCC CRL-1864 et la RF-48 ayant le numéro ATCC CRL-1863.

3. Méthode selon l'une quelconque des revendications 1 ou 2, dans laquelle le milieu est du Leibovitz L15 contenant 10 % (vol/vol) de sérum bovin foetal et 2 mM de L-glutamine, et dans laquelle la lignée cellulaire est cultivée à une température de 37° C en absence de CO2.

4. Méthode selon l'une quelconque des revendications 1 à 3, dans laquelle le milieu est changé au moins tous les 4 jours.

5. Méthode selon l'une quelconque des revendications 1 à 4, dans laquelle la lignée cellulaire est déposée et cultivée dans une plaque de culture après avoir obtenu une confluence cellulaire, dans laquelle la plaque est stockée dans les mêmes conditions d'incubation que celles utilisées pour cultiver la lignée cellulaire, et dans laquelle la production de ghréline est mesurée en utilisant un kit d'immuno-essai.

6. Méthode selon l'une quelconque des revendications 1 à 5, dans laquelle la lignée cellulaire est utilisée afin d'étudier l'expression génétique de la ghréline, de préférence par RT-PCR quantitative.

7. Méthode selon l'une quelconque des revendications 1 à 6, dans laquelle la lignée cellulaire est exposée à une variété de composés tests.

8. Méthode selon l'une quelconque des revendications 1 à 7, dans laquelle la méthode est une méthode pour développer les médicaments ou les ingrédients fonctionnels pour des produits alimentaires qui modulent les sensations de satiété ou d'appétit chez les êtres humains ou les animaux.

9. Utilisation d'une lignée cellulaire dérivée d'un adénocarcinome gastrique qui est capable de produire de la ghréline lorsqu'il est cultivé dans un milieu adapté, pour l'évaluation de (la régulation de) l'expression, de la synthèse et/ou de la libération de la ghréline in vitro.

10. Utilisation selon la revendication 9, dans laquelle la lignée cellulaire est sélectionnée parmi la RF-1 ayant le numéro ATCC CRL-1864 et la FR-48 ayant le numéro ATCC CRL-1863.
